# EUROPEAN PATENT APPLICATION

(11) **EP 2 799 140 A2**
(43) Date of publication of application: **05.11.2014**
(21) Application number: 14166805.3
(22) Date of filing: 01.05.2014
(51) Int. Cl.: B01L 3/00, G01N 1/28, G01N 33/50

(54) **Apparatus and method for the immunocamouflage of biological cells**

(30) Priority: 02.05.2013 US 201313875555
(71) Applicant: Canadian Blood Services, Ottawa, Ontario K1G 4J5 (CA)
(72) Inventor: Scott, Mark D., Surrey, British Columbia V4A 9R8 (CA)
(74) Representative: Roberts, Mark Peter

(57) **Abstract**

A device and method for polymer modification of biological cells includes pumping a first liquid mixture comprising biological cells from a first reservoir to a mixing chamber and a second liquid mixture comprising activated polymer from a second reservoir to the same mixing chamber, independently controlling an output volume of the first and second liquid mixtures pumped into the mixing chamber using at least one pump, mixing the first and second liquid mixtures in the mixing chamber to produce a final mixture comprising polymer-modified biological cells, and evacuating the final mixture comprising polymer-modified biological cells from the mixing chamber into an output reservoir.

## Description

### TECHNICAL FIELD

The present invention relates generally to apparatus and method for polymer modification of biological cells, and more particularly relates to the immunocamouflage of biological cells.

### BACKGROUND

Polymers such as methoxypolyethylene glycol (mPEG), hyperbranched polyglycerols (HPG) and polyoxasolines (POZ) are known for their non-toxic properties. In the field of immunology, these polymer classes are particularly useful for improving biocompatibility and reducing immunological recognition of cells when it is covalently grafted to cell surfaces.

Most common techniques for grafting polymers to biological cells involve manually mixing a biological cell solution and an activated polymer in buffer and then agitating the resulting mixture to obtain polymer-modified cells. This manual (i.e. non-automated) mixing method usually results in a high hydrolysis rate of activated polymer, poor homogeneity of polymer-modified cells product, low control of the mixture's sterility, in addition to being relatively slow, time consuming and unsuitable for scaling up to process larger volumes.

Accordingly there remains a need for an improved device and method for grafting polymers to biological cells.

### SUMMARY

In accordance with one aspect of the present invention, there is provided a device for grafting polymers to biological cells to immunocamouflage the biological cells comprising: a first reservoir containing a first liquid mixture of biological cells; a second reservoir containing a second liquid mixture of activated polymer; at least one pump in fluid flow communication with both the first and second reservoirs and which respectively transfers the first and second liquid mixtures from the first and second reservoirs into a common mixing chamber, the pump being operable to independently control an output volume of each of the first and second liquid mixtures fed from each of the first and second reservoirs into the mixing chamber; the mixing chamber being in fluid flow communication with the at least one pump and receiving therein said output volumes of the first and second liquid mixtures, the output volumes mixing within the mixing chamber in a predetermined volume ratio to produce a final mixture comprising polymer-modified biological cells; and an outlet for evacuating the final mixture of said polymer-modified biological cells from the mixing chamber.

There is also provided, in accordance with another aspect of the present invention, a method for producing polymer-modified biological cells comprising the steps of: pumping a first liquid mixture comprising biological cells from a first reservoir to a mixing chamber and pumping a second liquid mixture comprising activated polymer from a second reservoir to said mixing chamber; independently controlling a predetermined output volume of the first and second liquid mixtures pumped into the mixing chamber; mixing the predetermined output volumes of the first and second liquid mixtures in the mixing chamber to produce a final mixture ratio comprising polymer-modified biological cells; and evacuating the final mixture comprising the polymer-modified biological cells from the mixing chamber into an output reservoir.

### BRIEF DESCRIPTION OF THE DRAWINGS

Further features and advantages will become apparent from the following detailed description, taken in combination with the appended drawings, in which:
Fig. 1 is a schematic view of a device in accordance with one embodiment of the present disclosure which comprises a syringe pump;
Fig. 2 is a schematic view of a device in accordance with another embodiment of the present disclosure which comprises at least one peristaltic pump for actuating the output flow of a first and second reservoirs;
Fig. 3 is a schematic view of a device in accordance with a further embodiment of the present disclosure which comprises multiple peristaltic pumps each independently controlling the flow of different mixtures;
Fig.4 is a flow-chart illustrating the steps of a method for pegylating biological cells in accordance with an embodiment of the present disclosure; and
Fig. 5 is a schematic diagram showing a possible range of homogeneity of biological cells and polymers and the controlled narrow desired grafting range that is possible with the present device.

### DETAILED DESCRIPTION

The present disclosure relates generally to devices and methods for grafting polymers to biological cells such as to produce biological cells having covalently grafted polymers (i.e. "polymer-grafted" or "polymer-modified" cells), a process which is referred to as immunocamouflage of the biological cells. The process for grafting polymers to biological cells requires a biological cell solution to be mixed with an activated polymer, such as to subsequently obtain polymer-modified cells. The activated polymers used in conjunction with the devices and methods of the present disclosure may comprise, but are not limited to, methoxypolyethylene glycol (mPEG), hyperbranched polyglycerol and/or polyoxasolines. These activated polymers have chemical linker groups and may comprise a number of different activation chemistries. Linker molecules may comprise, inter alia, cyanuric chloride, imidazolyl formate, succinimidyl succinate, succinimidyl carbonate, succinimidyl glutarate, N-hydroxysuccinimide, 4-nitrophenol, and 2,4,5-trichlorophenol. The linker molecules listed above are exemplary only. Any linker molecule capable of covalently attaching to the polymer may be similarly used.

The devices and systems for the immunocamouflage of biological cells of the present disclosure comprise generally a first reservoir containing a first liquid mixture of biological cells and a second reservoir comprising a second liquid mixture of one or more activated polymer (as exemplified by methoxypolyethylene glycol (mPEG), hyperbranched polyglycerol or polyoxasolines, for example). The first and second liquid reservoirs are in fluid communication with at least one fluid transfer device in the form of a pump which may have a variable and controllable output. The pump(s) respectively transfer(s) the first and second mixtures from the first and second reservoirs into a common mixing chamber. The fluid transfer device / pump independently controls at least one flow characteristic from each of the first and second reservoirs, including output volume, mass flow, flow rate, etc. The pump(s) thereby controls, for example, the volume of the first and second liquid mixtures received within the mixing chamber, such that these mixtures are mixed at a predetermined volume ratio. By mixing the first and second liquid mixtures at a predetermined volume ratio, a more homogenous and reproducible final mixture of polymer-modified biological cells is obtained. An outlet for evacuating the final mixture, comprising the polymer-modified biological cells, from the mixing chamber may also be provided.

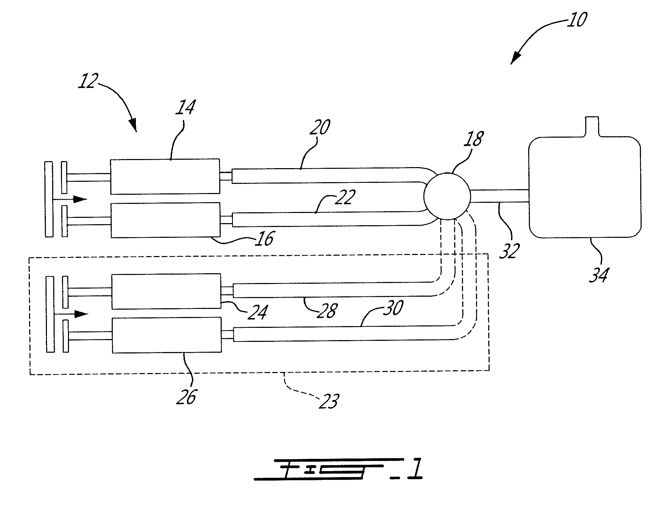

Referring to Fig. 1, the device 10 for the immunocamouflage of biological cells by polymer grafting (i.e. the device for grafting polymers to biological cells) in accordance with one embodiment of the present disclosure comprises a syringe pump 12 as the fluid transfer device which is disposed upstream of the common mixing chamber 18 fed by the syringe pump 12, as will be seen. The syringe pump 12 comprises at least one first syringe 14 defining a first reservoir which comprises a first liquid mixture of biological cells and at least one second syringe 16 defining a second reservoir which comprises a second liquid mixture of activated polymer (which may include, for example, methoxypolyethylene glycol (mPEG), hyperbranched polyglycerol (HPG), or polyoxasolines).. As illustrated in Fig. 1, the syringes 14, 16 are each connected in fluid communication with a mixing chamber 18 by tubes 20, 22 respectively. Although not depicted, the syringes 14, 16 may also be directly connected in fluid communication with the mixing chamber 18 thereby avoiding the use of the intermediary tubes 20, 22. The syringes 14, 16 allow for the independent control of the output volume of each of the first and second liquid mixtures therefrom, and therefore received and mixed within the mixing chamber 18.

In accordance with one embodiment, the ratio of biological cells and activated polymer micro-mixing in the mixing chamber 18 is controlled by the volumes of the first and second liquid mixtures pumped by the syringes 14, 16 at fixed concentrations.

The syringes 14, 16 may therefore be configured to pump a substantially equal volume of the first and second liquid mixtures. For example, 60 mL syringes can be used for each syringe 14, 16. Alternatively, different sizes of syringes may be used but each would be filled with a substantially equal volume of the first and second liquid mixtures.

In another embodiment, the output volume of the first and second liquid mixtures may be different and can be controlled by using different syringe volumes or different volumes of liquid mixtures within the syringes of the same size.

Alternatively still, as shown in Fig. 1, the ratio of biological cells and activated polymer micro-mixing in the mixing chamber 18 may be altered by adding an additional, optional, pump component 23 which may for example include additional syringes 24 and 26. Each of the additional syringes 24, 26 are in fluid communication with the same mixing chamber 18, for example via tubes 28, 30. Each additional syringe 24, 26 can pump the first liquid mixture of biological cells, the second liquid mixture of activated polymer(s) (of similar or different composition as to that provided by the syringe 16, and which may have, for example, different polymer molecular weights and/or different polymer species, as exemplified by using both mPEG and HPG to modify the cells) or other solutes to alter the volume ratio of biological cells and activated polymer which mix within the mixing chamber 18. Examples of other solutes include, but are not limited to, isotonic saline, phosphate buffered saline solutions of variable pH; or other isotonic buffers. Therefore, in this particular configuration of the device 10, the syringe pump 12 can comprises 4 syringes, however any number of syringes greater than or equal to 2, can be used.

It is known that when a solution of chemically activated polymers is prepared in a large volume, a high hydrolysis rate of the activation chemistry is observed, which results in an inactivation of the polymer and therefore reduced time-dependent covalent grafting to biological cells resulting in a lower homogeneity of polymer grafted biological cells. The embodiment illustrated in Fig.1 allows the hydrolysis rate of the activated polymers to be controlled, as the activated polymer is prepared in small batches.

The device 10 further comprises an outlet conduit 32 in fluid communication with the mixing chamber 18 for evacuating the final mixture comprising polymer-modified biological cells from the mixing chamber 18. As illustrated in Fig.1, the outlet may be in fluid communication with a final reservoir 34 used to store the polymer-grafted biological cells.

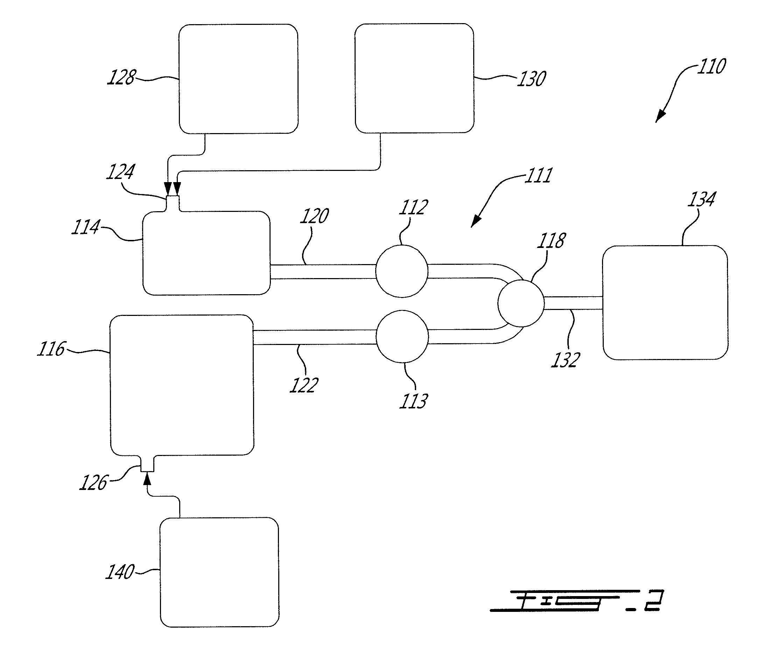

Referring now to the alternate embodiment of Fig. 2, the device 110 for the immunocamouflage of biological cells by polymer grafting (i.e. a device for grafting polymers to biological cells) includes a pump or fluid transfer device 111 which in this embodiment comprises at least one peristaltic pump in serial flow with the solution input lines 120 and 122 feeding the mixing chamber 118, and more particularly intermediately positioned between the upstream first and second reservoirs 114, 116 and the downstream common mixing chamber 118. In the depicted embodiment, two peristaltic pumps 112, 113 are in fact provided, each of which is in fluid communication with the first and second reservoirs 114, 116 and the mixing chamber 118. The peristaltic pumps 112, 113 independently control the output volume of the first and second liquid mixtures from the first and second reservoirs 114, 116 to the mixing chamber 118. Although not illustrated here, the fluid transfer device 111 may also comprise a single peristaltic pump for simultaneously controlling the output volume from the first and second reservoirs 114, 116 into the common mixing chamber 118. As per the syringes 14, 16 of the syringe pump forming the fluid transfer device 12 described above, the peristaltic pump(s) 112, 113 of the fluid transfer device 111 is/are operable to control at least the volume of the first and second liquid mixtures (i.e. the solution of biological cells and the solution of activated polymers) independently, such that these mixtures are mixed at a predetermined volume ratio. As a result, a more homogenous and reproducible final mixture of polymer-modified biological cells is thereby obtained.

While it may be possible to directly connect the peristaltic pumps 112, 113 to the first and second reservoirs 114, 116, in the depicted embodiment of the device 110 a first tube 120 is in fluid communication with the first reservoir 114 and the mixing chamber 118 and a second tube 122 is in fluid communication with the second reservoir 116 and the mixing chamber 118, with the first peristaltic pump 112 being in-line with the first tube 120 and the second peristaltic pump 113 being in-line with the second tube 130. In alternative embodiments, other piping and/or tubing configurations may be employed, provided that the peristaltic pumps 112, 113 are disposed in-line between each of the reservoirs 114, 116 and the common mixing chamber 118.

The device 110 for the immunocamouflage of biological cells by polymer grafting of Fig.2 is a substantially continuous flow device, given that the pumps 112, 113 are operable to continuously draw solution from the first and second reservoirs 114, 116, respectively, for pumping into the common mixing chamber 118. The first and second reservoirs 114, 116 respectively have docking ports 124 and 126, such as to enable the continuous re-filling of the first and second liquid mixtures within the first and second reservoirs 114, 116. This may be done using external reservoirs 128, 130 and 140. Although the device 110 may not include such additional external reservoirs 128, 130 and 140, in one particular embodiment they are provided in order to further enable continuous operation of the system. More particularly, as seen in Fig. 2, a buffer reservoir 128 and a cell reservoir 130 which feed the first reservoir 114 via port 124, and a polymer reservoir 140 which feed the second reservoir 116 via the portion 126. The buffer reservoir 128 may contain, in one particular embodiment for example, a red blood cell dilution buffer having a pH of approximately 8, and the cell reservoir 130 may comprise a red blood cell bag containing 1 unit of blood. The polymer reservoir 140 may contain an activated polymer solution which is fed through the port 126 into the second reservoir 116. In another alternate embodiment, the polymer reservoir 140 feeding the second reservoir 116 may include a buffering solution into which is mixed the polymer in anhydrous powder form, to create the activated polymer solution. The polymer powder may be contained in a separate external reservoir or may be mixed into the extern buffer reservoir 140.

In one possible embodiment of the device 110, one or more additional peristaltic pumps are provided to feed the solution into the first and second reservoirs 114, 116. These may be provided, for example, in line between the external reservoirs 128 and 130 and the first reservoir 114 (either a single peristaltic pump for both external reservoir feeds or one for each), and/or between the external reservoir 140 and the second reservoir. The device 110 operates substantially continuously, and has the added advantage of having few disruptions in mixtures sterility and providing a homogenous pegylated biological cells mixture.

In order to control the ratio of the first and second liquid mixtures micro-mixing within the mixing chamber 118, the cross-sectional area of the first tube 120 and the second tube 122 may be the same or different, as required. When the cross-sectional area of tubes 120 and 122 is the same, a substantially equal volume of the first and second liquid mixture is accordingly pumped into and mixed within the mixing chamber 118, assuming that the first and second peristaltic pumps 112, 113 are operating at a common rate. Given the same conditions, when the cross-sectional area of tubes 120 and 122 is different, a substantially unequal volume of the first and second liquid mixture is fed into and mixed within the mixing chamber 118.

When two peristaltic pumps 112, 113 are provided such as to independently control the output volume from the first and second reservoirs 114, 116 respectively, the volume ratio of the first and second liquid mixtures may also be different and varied, and thus controlled, by actuating the peristaltic pumps 112, 113 at different rotational speeds and thus at different output flow rates.

The mixing chamber 118 of the device 110 further comprises an outlet conduit 132 in fluid communication with both the mixing chamber 118 and a downstream a final reservoir 134, for evacuating the final solution mixture comprising polymer-modified biological cells from the mixing chamber 118.

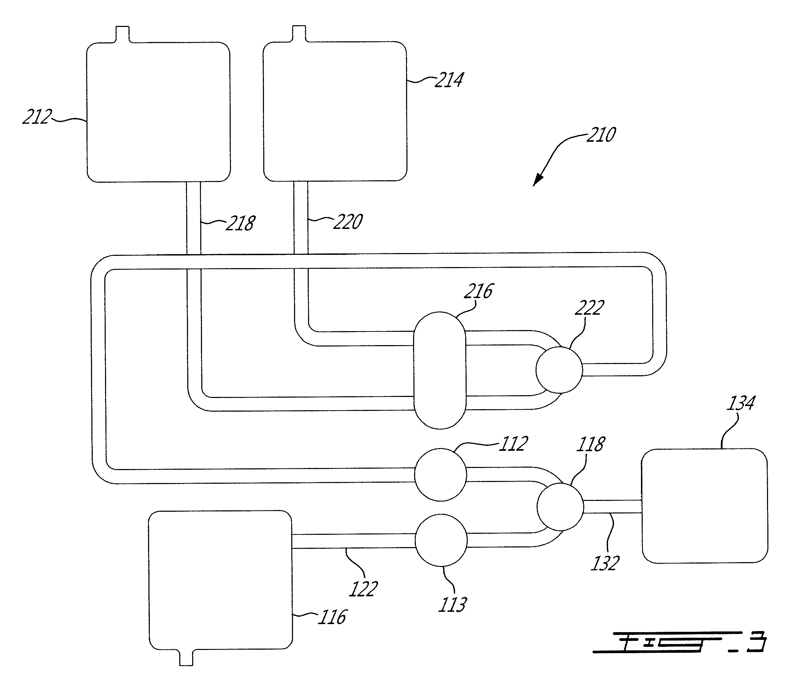

Referring now to Fig. 3, an alternative way to control the homogeneity of the final mixture and to continuously produce same is to pre-mix a solution of buffer and a liquid mixture of polymer-modified biological cells is shown. The device 210 comprises a third reservoir 212 containing a buffer and a forth reservoir 214 containing biological cells. The third and forth reservoirs 212, 214 are independently in fluid communication with at least one additional peristaltic pump 216 via tubes 218, 220. The additional peristaltic pump 216 controls the output flow of both the buffer and biological cell reservoirs 212, 214 which is fed into a preliminary mixing chamber 222, thereby producing an output from this preliminary mixing chamber 222 in which is a diluted liquid mixture comprising biological cells. The output flow of the preliminary mixing chamber 222 is in turn controlled by a downstream peristaltic pump 112 in similar manner to that described above in connection with the embodiment of Fig.2.

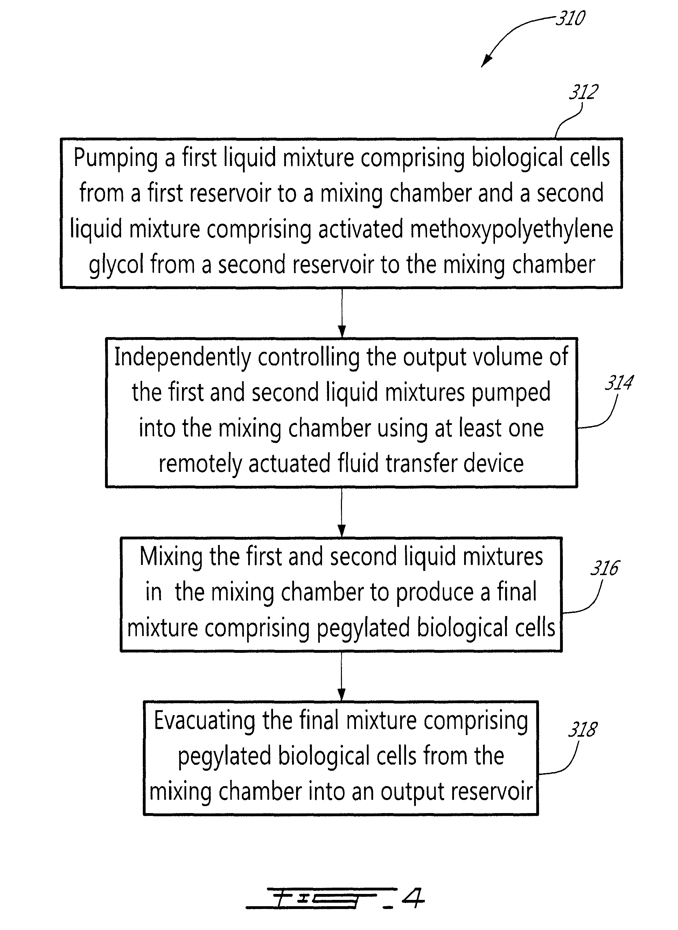

Referring to Fig. 4, the present disclosure further comprises a method 310 for the immunocamouflage of biological cells by polymer grafting, i.e. for the production of polymer-modified biological cells. The method comprises a first step 312 of pumping a first liquid mixture comprising biological cells from a first reservoir to a mixing chamber and pumping a second liquid mixture comprising activated polymer (as exemplified by methoxypolyethylene glycol (mPEG), hyperbranched polyglycerol, or polyoxasolines) from a second reservoir to the same mixing chamber. The output volume of the first and second liquid mixtures is then pumped into the mixing chamber are independently controlled using at least one remotely actuated fluid transfer device 314. The method further comprises the step 316 of mixing the first and second liquid mixtures in the mixing chamber to produce a final mixture comprising polymer-modified biological cells. This mixing within the mixing chamber may include either passive mixing, forced only by the pumped input of solutions or may alternately be active mixing aided by an automatically operating (i.e. not manual) mixing mechanism. In either case, the fluid transfer device being used may be controlled and actuated remotely such that it operates autonomously and continuously. The final method step 318 includes evacuating the final mixture comprising polymer-modified biological cells from the mixing chamber into an output reservoir.

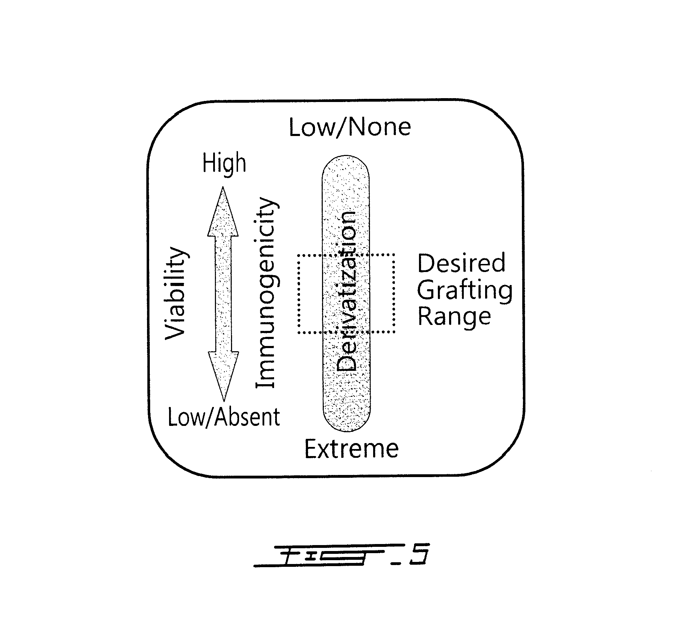

The presently described device enables the control and maintenance of the homogeneity of the ratio/mix of biological cells (such as red blood cells, for example) and the polymer (such as mPEG, for example) within a desired narrow band best suited for polymer grafting. As seen in Fig. 5, a possible range of homogeneity of biological cells and the polymer is schematically depicted, and the narrow desired grafting range made possible with the present device is shown within this range of mixing ration of polymer to biological cells. The present devices enable a production method of polymer-modified cell solution having a substantially constant ratio within the desired grafting range.

The polymer-modified cells described herein may include, but are not limited to, red blood cells, platelets, white blood cells, or any suspension of cells or cell aggregates such as pancreatic islets, and the like.

The embodiments of the invention described above are intended to be exemplary. Those skilled in the art will therefore appreciate that the forgoing description is illustrative only, and that various alternatives and modifications can be devised without departing from the spirit of the present invention. Accordingly, the present is intended to embrace all such alternatives, modifications and variances which fall within the scope of the appended claims.

## Claims

1. A device for grafting polymers to biological cells to immunocamouflage the biological cells comprising:
a first reservoir containing a first liquid mixture of biological cells;
a second reservoir containing a second liquid mixture of activated polymer;
at least one pump in fluid flow communication with both the first and second reservoirs and which respectively transfers the first and second liquid mixtures from the first and second reservoirs into a common mixing chamber, the pump being operable to independently control an output volume of each of the first and second liquid mixtures fed from each of the first and second reservoirs into the mixing chamber;
the mixing chamber being in fluid flow communication with the at least one pump and receiving therein said output volumes of the first and second liquid mixtures, the output volumes mixing within the mixing chamber in a predetermined volume ratio to produce a final mixture comprising polymer-modified biological cells; and
an outlet for evacuating the final mixture of said polymer-modified biological cells from the mixing chamber.

2. The device according to claim 1, wherein the second liquid of activated polymer comprises at least one of: methoxypolyethylene glycol (mPEG), hyperbranched polyglycerol, andpolyoxasolines.

3. The device according to claim 1 or 2, wherein the at least one pump comprises a syringe pump, preferably automated for reciprocal operation, having at least one first syringe defining the first reservoir and pumping said first liquid mixture of biological cells and at least one second syringe defining the second reservoir and pumping the second liquid mixture of activated polymer, the syringe pump optionally having more than two syringes.

4. The device according to claim 3, wherein the first and second syringes defining the first and second reservoirs contain a substantially equal volume of the first and second liquid mixtures respectively, thereby pumping the same volume ratio of both liquid mixtures.

5. The device according to claim 3, wherein the syringe defining the first reservoir contains a substantially unequal volume of the first liquid mixture compared to the volume of the second liquid mixture contained in the syringe defining the second reservoir, the first and second syringes thereby pumping a different volume ratio of the first and second liquid mixtures into the mixing chamber.

6. The device according to any one of the preceding claims, wherein the at least one pump comprises at least one peristaltic pump operable to control the output volume of the first and second reservoirs, the at least one peristaltic pump being in-line between the first and second reservoirs and the mixing chamber.

7. The device according to claim 6, further comprising a first tube in fluid communication with the first reservoir and the mixing chamber and a second tube in fluid communication with the second reservoir and the mixing chamber, each of the first and second tubes extending through the at least one peristaltic pump and extending substantially uninterrupted from the first and second reservoirs to the mixing chamber.

8. The device according to claim 6, further comprising a first set of tubes having a first tube in fluid communication with the first reservoir and the peristaltic pump and a second tube being in fluid communication with the peristaltic pump and the mixing chamber, and a second set of tubes having first tube being in fluid communication with the second reservoir and the peristaltic pump and a second tube being in fluid communication with the peristaltic pump and the mixing chamber.

9. The device according to claim 8, wherein the cross-sectional area of the first and second set of tubes is the same such as to provide a substantially equal volume of the first and second liquid mixtures to the mixing chamber.

10. The device according to claim 8, wherein the cross-sectional areas of the first and second set of tubes are different, thereby providing a substantially unequal volume of the first and second mixtures to the mixing chamber.

11. The device according to any one of claims 6 to 10, wherein the at least one pump comprises either a single peristaltic pump which simultaneously controls the output volume from both the first and second reservoirs, or two peristaltic pumps each independently controlling the output volume from the first and second reservoirs respectively, the two peristaltic pumps being optionally actuated at different speeds for providing a substantially unequal volume of the first and second liquid mixtures to the mixing chamber.

12. A method for producing polymer-modified biological cells comprising the steps of:
pumping a first liquid mixture comprising biological cells from a first reservoir to a mixing chamber and pumping a second liquid mixture comprising activated polymer from a second reservoir to said mixing chamber;
independently controlling a predetermined output volume of the first and second liquid mixtures pumped into the mixing chamber;
mixing the predetermined output volumes of the first and second liquid mixtures in the mixing chamber to produce a final mixture ratio comprising polymer-modified biological cells; and
evacuating the final mixture comprising the polymer-modified biological cells from the mixing chamber into an output reservoir.

13. The method of claim 12, further comprising providing the second liquid mixture of activated polymer, the activated polymer comprising at least one of: methoxypolyethylene glycol (mPEG), hyperbranched polyglycerol, and polyoxasolines.

14. The method of claim 12 or 13, wherein the step of independently controlling the output volume of the first and second liquid mixtures further comprises using at least one remotely actuated syringe pump having at least a first syringe defining the first reservoir and at least a second syringe defining the second reservoir, optionally including remotely controlling the syringe pump autonomously.

15. The method of claim 12 or 13, wherein the step of independently controlling the output volume of the first and second liquid mixtures further comprises using at least one remotely actuated peristaltic pump, optionally including remotely controlling the peristaltic pump autonomously.
